# EUROPÄISCHE PATENTANMELDUNG

(11) **EP 4 018 960 A1**
(43) Veröffentlichungstag der Anmeldung: **29.06.2022**
(21) Anmeldenummer: 21211208.0
(22) Anmeldetag: 30.11.2021
(51) Int. Cl.: A61B 90/70, A61M 1/00, A61B 17/29

(54) **HANDHABUNGSEINRICHTUNG**

(30) Priorität: 22.12.2020 DE 102020134601
(71) Anmelder: Karl Storz SE & Co. KG, 78532 Tuttlingen (DE)
(72) Erfinder: BLOCHER, Martin, 78532 Tuttlingen (DE); GRÜNER, Sven Axel, 78532 Tuttlingen (DE); HOLZER, Judith, 78532 Tuttlingen (DE); KÄRCHER, Daniel, 78532 Tuttlingen (DE); LÄNGLE, Dominik, 78532 Tuttlingen (DE); MERZ, Robin, 78532 Tuttlingen (DE); SCHNEIDER, Janosz, 78532 Tuttlingen (DE); SCHNEIDER, Sven, 78532 Tuttlingen (DE); STEFAN, Jochen, 78532 Tuttlingen (DE); UNGER, Tobias, 78532 Tuttlingen (DE)

(57) **Zusammenfassung**

Eine Handhabungseinrichtung (14) für ein mikroinvasives medizinisches Instrument (10) umfasst einen ersten Griffschenkel (22) und einen zweiten Griffschenkel (40), die relativ zu einander bewegbar sind, einen Spülanschluss (90) zum Empfangen einer Spülflüssigkeit zur Reinigung der Handhabungseinrichtung (14) zwischen zwei Verwendungen und einen Spülkanal (92, 94, 96, 98), der den Spülanschluss (90) mit einem oder mehreren Ausgängen (93, 95, 33, 65) verbindet. Der Spülkanal (92, 94, 96, 98) ist in dem ersten Griffschenkel (22) angeordnet.

## Beschreibung

Die vorliegende Erfindung ist auf eine Handhabungseinrichtung für ein mikroinvasives medizinisches Instrument und ein mikroinvasives medizinisches Instrument mit einer solchen Handhabungseinrichtung bezogen.

Viele mikrochirurgische Instrumente sind arretierbar. Dies gilt insbesondere für mikrochirurgische Instrumente mit Greifwerkzeugen an ihren distalen Enden. Die Arretierung erfolgt fast ausschließlich an der Handhabungseinrichtung am proximalen Ende, und zwar kraftschlüssig oder - häufiger - formschlüssig. Beispielsweise ist an einem von zwei relativ zueinander bewegbaren Griffschenkeln ein Rastsporn mit mehreren oder zahlreichen Rastnuten oder Rastzähnen vorgesehen, am anderen Griffschenkel eine Rastklinke, die in eine beliebige der Rastnuten eingreifen oder einen beliebigen der Rastzähne hintergreifen kann.

Zum Lösen der Arretierung ist in der Regel eine Löseeinrichtung vorgesehen, beispielsweise ein Lösehebel, der unmittelbar manuell betätigbar, nämlich bewegbar ist, um die Rastklinke aus ihrer Rastposition, in der sie mit dem Rastsporn form- und/oder kraftschlüssig verbunden ist, in eine Löseposition ohne Eingriff bewegt. Hinzukommen kann eine Abschalteinrichtung, um die Arretiereinrichtung dauerhaft außer Eingriff zu halten. Die gesamte Mechanik zur Arretierung weist einen komplexen Aufbau und eine komplexe Geometrie auf. Entsprechend aufwendig kann eine vollständige Reinigung sein, die bei einem wiederverwendbaren Instrument vor der Sterilisation erfolgen muss.

Eine Aufgabe der vorliegenden Erfindung besteht darin, eine verbesserte Handhabungseinrichtung für ein medizinisches Instrument zu schaffen.

Diese Aufgabe wird gelöst durch die Gegenstände der unabhängigen Ansprüche.

Weitere Ausführungsformen sind in den abhängigen Ansprüchen definiert.

Eine Handhabungseinrichtung für ein medizinisches Instrument umfasst einen ersten Griffschenkel und einen zweiten Griffschenkel, die relativ zu einander bewegbar sind, einen Spülanschluss zum Empfangen einer Spülflüssigkeit zur Reinigung der Handhabungseinrichtung zwischen zwei Verwendungen und einen Spülkanal, der den Spülanschluss mit einem oder mehreren Ausgängen verbindet, wobei der Spülkanal in dem ersten Griffschenkel angeordnet ist.

Die Handhabungseinrichtung ist insbesondere vorgesehen und ausgebildet für ein mikroinvasives medizinisches Instrument, als für ein Instrument, das seinerseits für eine Verwendung bei mikroinvasiven medizinischen Maßnahmen vorgesehen und ausgebildet ist. Die Handhabungseinrichtung ist insbesondere wiederverwendbar, kann also wiederholt gereinigt und sterilisiert, insbesondere autoklaviert werden.

Die Handhabungseinrichtung kann integraler Bestandteil eines medizinischen Instruments, sein. Dies bedeutet, dass die Handhabungseinrichtung mit einem oder mehreren weiteren Bestandteilen des Instruments dauerhaft und derart verbunden ist, dass eine Trennung nicht ohne Verwendung von Werkzeug oder überhaupt nicht reversibel, also zerstörungsfrei möglich ist.

Alternativ kann die Handhabungseinrichtung Bestandteil eines medizinischen Instruments, aber von weiteren Bestandteilen des mikroinvasiven medizinischen Instruments ohne Weiteres, insbesondere ohne Werkzeug und zerstörungsfrei, das heißt reversibel trennbar und mit diesen wieder verbindbar sein.

Der Spülanschluss ist insbesondere ein LUER-Anschluss, das heißt ein konkaver LUER-Konus gemäß ISO 80369 bzw. DIN EN 80369-7. In diesem Fall weist er eine Länge von maximal 7,5 mm, eine Steigung von 6 Prozent, das heißt einen Winkel von 3,44 Grad, und einen minimalen Durchmesser von 4 mm auf. Durch den Spülanschluss kann Spülflüssigkeit mit einem Druck von beispielsweise circa 2 bar in den Spülkanal eingeleitet werden.

Der Spülkanal ist insbesondere verzweigt mit mehreren Abschnitten, die jeweils zu einem oder mehreren Ausgängen führen, durch die die Spülflüssigkeit aus dem ersten Griffschenkel austreten kann. Der Spülkanal kann häufig wechselnde Querschnitte aufweisen. Durch seitliche Öffnungen der Querschnitte kann Spülflüssigkeit aus dem Spülkanal in angrenzende Bereiche des ersten Griffschenkels übertreten.

Die Zufuhr von Spülflüssigkeit zu einem Spülkanal, durch den die Spülflüssigkeit in das Innere des ersten Griffschenkels geleitet werden kann, kann eine besonders wirksame Reinigung im Inneren des ersten Griffschenkels und vor allem auch an den Stellen, an denen die Spülflüssigkeit wieder aus dem ersten Griffschenkel austritt, ermöglichen. Ein an von innen nach außen gerichteter Strom von Spülflüssigkeit kann Verunreinigungen besonders wirksam aus dem ersten Griffschenkel entfernen.

Bei einer Handhabungseinrichtung, wie sie hier beschrieben ist, ist der Spülanschluss insbesondere an einer dem zweiten Griffschenkel zugewandten Seite des ersten Griffschenkels angeordnet.

Die einander zugewandten Seiten oder Oberflächenbereiche der Griffschenkel werden bei der Verwendung der Handhabungseinrichtung oft weniger verunreinigt als die voneinander abgewandten Seiten, also die nach außen orientierten Oberflächenbereiche der Griffschenkel. Da der Spülanschluss selbst eine Öffnung darstellt, in der und durch die hindurch eine Verunreinigung möglich ist, kann die Anordnung des Spülanschlusses an der dem zweiten Griffschenkel zugewandten Seite des ersten Griffschenkels die Verunreinigung des ersten Griffschenkels durch den Spülanschluss hindurch reduzieren.

Bei einer Handhabungseinrichtung, wie sie hier beschrieben ist, ist der Spülanschluss insbesondere proximal eines Verbindungsbereichs der Griffschenkel angeordnet.

Der Verbindungsbereich der Griffschenkel ist insbesondere der Bereich, in dem die Griffschenkel durch ein Gelenk miteinander mechanisch verbunden sind. Die Anordnung des Spülanschlusses proximal des Verbindungsbereichs bedeutet, dass hinsichtlich des Pfads, entlang dessen Kräfte und Momente von dem Griffschenkel zu einem distalen Bereich, beispielsweise zu einem Schaft und einem Werkzeug am distalen Ende des mikroinvasiven medizinischen Instruments übertragen werden, der Verbindungsbereich zwischen dem Spülanschluss und dem Schaft angeordnet ist. Der Spülanschluss ist somit in dem Bereich angeordnet, in dem typischerweise auch eine Arretiereinrichtung zum Arretieren der Griffschenkel relativ zueinander vorgesehen ist.

Bei einer Handhabungseinrichtung, wie sie hier beschrieben ist, ist der Spülkanal insbesondere gegabelt und verbindet fluidisch den Spülanschluss mit mehreren Ausgängen.

Der Spülkanal kann also nach einer oder mehreren Verzweigungen mehrere hinsichtlich des Fluidstroms parallel geschaltete Abschnitte aufweisen, durch die eine Spülflüssigkeit verschiedene Bereiche des ersten Griffschenkels erreichen kann.

Bei einer Handhabungseinrichtung, wie sie hier beschrieben ist, ist der zweite Griffschenkel relativ zu dem ersten Griffschenkel insbesondere in einer Ebene bewegbar, wobei der Spülkanal im Wesentlichen parallel zu der Ebene verläuft.

Die Griffschenkel sind relativ zueinander insbesondere um eine durch ein Gelenk zwischen den Griffschenkeln definierte Schwenkachse schwenkbar. Die Ebene, in der der zweite Griffschenkel relativ zu dem ersten Griffschenkel bewegbar ist, ist dann orthogonal zu dieser Schwenkachse. Der Spülkanal ist im Wesentlichen parallel zu der Ebene, wenn zumindest die meisten Abschnitte des Spülkanals parallel zu der Ebene sind oder mit der Ebene einen Winkel einschließen, der nicht größer als 5 Grad oder nicht größer als 10 Grad ist. Durch den Spülkanal strömende Spülflüssigkeit weist also überwiegend Strömungsrichtungen parallel zu der Ebene oder in kleinem Winkel zu der Ebene auf.

Eine Handhabungseinrichtung, wie sie hier beschrieben ist, umfasst insbesondere ferner einen Arretierungssporn, der mit dem zweiten Griffschenkel mechanisch starr verbunden oder gekoppelt ist, sodass eine Bewegung des zweiten Griffschenkels relativ zu dem ersten Griffschenkel mit einer Bewegung des Arretierungssporns relativ zu dem ersten Griffschenkel einhergeht, eine Arretiereinrichtung, die mit dem ersten Griffschenkel mechanisch verbunden und relativ zu dem ersten Griffschenkeln bewegbar ist zwischen einer Löseposition ohne Wechselwirkung mit dem Arretierungssporn und einer Arretierungsposition, in der die Arretiereinrichtung form- oder kraftschlüssig mit dem Arretierungssporn verbunden sein kann, und eine Löseeinrichtung, die manuell bewegbar ist zwischen einer Löseposition und einer Arretierungsposition, und die derart mit der Arretiereinrichtung mechanisch gekoppelt ist, dass die Arretiereinrichtung ihre Löseposition einnimmt, wenn die Löseeinrichtung ihre Löseposition einnimmt, und dass die Arretiereinrichtung ihre Arretierungsposition einnimmt, wenn die Löseeinrichtung ihre Arretierungsposition einnimmt.

Der Arretierungssporn ist insbesondere ein Rastsporn mit mehreren Rastnuten oder mehreren Rastzähnen. Die Arretiereinrichtung umfasst insbesondere eine Rastklinke, die in eine der mehreren Arretierungsnuten eingreifen oder einen der mehreren Arretierungszähne hintergreifen kann. In der Arretierungsposition der Arretiereinrichtung kann die Arretiereinrichtung form- oder kraftschlüssig mit dem Arretierungssporn verbunden sein. Es kann einen Bereich von insbesondere weit geöffneten Positionen des zweiten Griffschenkels relativ zu dem ersten Griffschenkel geben, in dem der Arretierungssporn nicht an die Arretiereinrichtung heranreicht und die Rastklinke in keine Rastnut eingreift oder keinen Rastzahn hintergreift.

Die Arretiereinrichtung und der Arretierungssporn sind insbesondere so ausgebildet, dass in der Arretierungsposition der Arretiereinrichtung eine Entfernung der Griffschenkel voneinander form- oder kraftschlüssig unterbunden ist. Eine weitergehende Annäherung der Griffschenkel aneinander kann hingegen ermöglicht sein. Dies kann beispielsweise durch eine asymmetrische Gestaltung von Rastnuten und Rastklinke ermöglicht sein.

Die Löseeinrichtung umfasst insbesondere einen Lösehebel mit einem ersten Arm, der unmittelbar manuell betätigbar, nämlich bewegbar ist, und einem zweiten Arm, der mit der Arretiereinrichtung einstückig ausgebildet oder mit der Arretiereinrichtung mechanisch starr verbunden oder mit der Arretiereinrichtung mechanisch gekoppelt sein kann. Der Lösehebel ist insbesondere so ausgebildet und angeordnet, dass er sich durch den ersten Griffschenkel hindurch erstreckt, der unmittelbar manuell betätigbare erste Arm des Lösehebels an einer von dem zweiten Griffschenkel abgewandten Seite des ersten Griffschenkels und der mit der Arretiereinrichtung einstückig ausgebildete, mechanisch verbundene oder mechanisch gekoppelte Arm des Lösehebels an einer dem zweiten Griffschenkel zugewandten Seite des ersten Griffschenkels angeordnet ist.

Bei einer Handhabungseinrichtung, wie sie hier beschrieben ist, verbindet insbesondere ein erster Abschnitt des Spülkanals den Spülanschluss mit der Löseeinrichtung.

Dies bedeutet, dass ein Ende des ersten Abschnitts, ein erster Ausgang des Spülkanals an der Löseeinrichtung angeordnet ist. Der erste Abschnitt des Spülkanals verbindet den Spülanschluss insbesondere mit einem zur unmittelbaren manuellen Betätigung vorgesehenen und ausgebildeten Bereich der Löseeinrichtung.

Die Löseeinrichtung, insbesondere der zur unmittelbaren manuellen Betätigung vorgesehene Bereich der Löseeinrichtug, ist aufgrund der ständigen Berührung durch die oft mit Blut, anderen Körperflüssigkeiten oder Geweberesten verunreinigten Händen von medizinischem Personal in besonderem Maße einer Verunreinigung ausgesetzt. Eine Zufuhr von Spülflüssigkeit zu der Löseeinrichtung kann eine Reinigung der Löseeinrichtung ermöglichen.

Bei einer Handhabungseinrichtung, wie sie hier beschrieben ist, verbindet der erste Abschnitt des Spülkanals insbesondere den Spülanschluss mit einer Ausnehmung, die die Löseeinrichtung in ihrer Löseposition zumindest teilweise aufnimmt.

Die Ausnehmung ist insbesondere als Nut ausgebildet, deren Gestalt zu der Gestalt des unmittelbar manuell betätigbaren Bereichs der Löseeinrichtung korrespondiert. In der in Löseposition der Löseeinrichtung füllt der unmittelbar manuell betätigbare Bereich der Löseeinrichtung die Ausnehmung weitgehend oder vollständig aus, sodass nur ein schmaler Spalt verbleibt, durch den Spülflüssigkeit strömen kann. Ein Ausgang des Spülkanals ist insbesondere teilweise oder vollständig in der Ausnehmung angeordnet, sodass Spülflüssigkeit abhängig von der Position der Löseeinrichtung mehr oder weniger in die Ausnehmung hinein strömen kann.

Bei einer Handhabungseinrichtung, wie sie hier beschrieben ist, ist die Löseeinrichtung insbesondere ausgebildet als um eine Schwenkachse schwenkbarer Hebel mit einem unmittelbar manuell bewegbaren ersten Arm und einem zweiten Arm, der mit dem ersten Arm mechanisch starr verbunden und mit der Arretiereinrichtung mechanisch gekoppelt ist, wobei ein erster Ausgang des Spülkanals näher an der Schwenkachse der Löseeinrichtung als an dem freien Ende des ersten Arms der Löseeinrichtung angeordnet ist.

Die Anordnung des ersten Ausgangs des Spülkanals näher an der Schwenkachse als an dem freien Ende des ersten Arms der Löseeinrichtung kann eine Umleitung von Spülflüssigkeit zu anderen Bereichen der Handhabungseinrichtung, insbesondere des ersten Griffschenkels ermöglichen.

Bei einer Handhabungseinrichtung, wie sie hier beschrieben ist, lenkt die Löseeinrichtung abhängig von ihrer Position durch den ersten Abschnitt des Spülkanals strömende Spülflüssigkeit mehr oder weniger in einen zweiten Abschnitt des Spülkanals.

Bei einer Handhabungseinrichtung, wie sie hier beschrieben ist, lenkt die Löseeinrichtung abhängig von ihrer Position durch den ersten Abschnitt des Spülkanals strömende Spülflüssigkeit mehr oder weniger in einen zweiten Abschnitt des Spülkanals, wobei ein Querschnitt des zweiten Abschnitts des Spülkanals teilweise durch die Löseeinrichtung begrenzt ist.

Die Lenkungswirkung der Löseeinrichtung beruht insbesondere darauf, dass die Löseeinrichtung abhängig von ihrer Position einen Ausgang des ersten Abschnitts des Spülkanals mehr oder weniger verschließt. Der zweite Abschnitt des Spülkanals ist insbesondere an dem Ausgang oder nahe dem Ausgang mit dem ersten Abschnitt fluidisch verbunden, oder der erste Abschnitt geht an dem Ausgang in den zweiten Abschnitt des Spülkanals über.

Der zweite Abschnitt des Spülkanals schließt mit dem ersten Abschnitt des Spülkanals insbesondere einen Winkel ein, der im Bereich zwischen 60 Grad oder 80 Grad und 100 Grad oder 120 Grad liegt. Aus dem ersten Abschnitt in den zweiten Abschnitt des Spülkanals strömende Spülflüssigkeit ändert ihre Bewegungsrichtung also um einen Winkel, der zwischen 60 Grad oder 80 Grad und 100 Grad oder 120 Grad liegt.

Wenn die Löseeinrichtung die Öffnung am Ende des ersten Abschnitts des Spülkanals nicht oder nur teilweise verschließt, kann durch den ersten Abschnitt des Spülkanals fließende Spülflüssigkeit insbesondere geradeaus oder im Wesentlichen geradeaus durch den Ausgang aus der Handhabungseinrichtung austreten oder in die Ausnehmung für die Löseeinrichtung oder deren unmittelbar manuell betätigbaren Arm übertreten. Wenn die Löseeinrichtung den Ausgang teilweise oder vollständig verschließt, wird durch den ersten Abschnitt des Spülkanals strömende Spülflüssigkeit teilweise oder vollständig in den zweiten Abschnitt des Spülkanals umgelenkt.

Die Lenkungswirkung der Löseeinrichtung kann eine Variation der Strömung der Spülflüssigkeit durch manuelle Betätigung der Löseeinrichtung ermöglichen. Durch den Spülanschluss zugeführte Spülflüssigkeit kann somit abwechselnd unterschiedlichen Bereichen der Handhabungseinrichtung zugeführt werden.

Indem der Querschnitt des zweiten Abschnitts des Spülkanals teilweise durch die Löseeinrichtung begrenzt ist, kann die Löseeinrichtung durch die Spülflüssigkeit umströmt und somit gereinigt werden. Der Querschnitt des zweiten Abschnitts des Spülkanals ist insbesondere zumindest teilweise durch den zweiten Arm der Löseeinrichtung begrenzt.

Bei einer Handhabungseinrichtung, wie sie hier beschrieben ist, leitet der zweite Abschnitt des Spülkanals Spülflüssigkeit insbesondere zu einem Verbindungsbauteil, das den zweiten Arm der Löseeinrichtung mit der Arretiereinrichtung mechanisch koppelt oder verbindet.

Das Verbindungsbauteil koppelt insbesondere ein von der Schwenkachse der Löseeinrichtung abgewandtes Ende des zweiten Arms der Löseeinrichtung mit der Arretiereinrichtung. Das Verbindungsbauteil ist insbesondere durch je ein Gelenk mit dem zweiten Arm der Löseeinrichtung und mit der Arretiereinrichtung mechanisch verbunden. Das Verbindungsbauteil ist bei der vorgesehenen Verwendung der Handhabungseinrichtung insbesondere ausschließlich auf Zug beansprucht.

Indem der zweite Abschnitt des Spülkanals Spülflüssigkeit zu dem Verbindungsbauteil leitet, kann auch dieses, gegebenenfalls einschließlich der Gelenke, mittels derer es mit der Löseeinrichtung und mit der Arretiereinrichtung verbunden ist, von Spülflüssigkeit umspült und gereinigt werden.

Bei einer Handhabungseinrichtung, wie sie hier beschrieben ist, ist der Spülanschluss insbesondere ferner mit einem Raumbereich zum Aufnehmen des Arretierungssporns fluidisch verbunden.

Insbesondere geht der erste Abschnitt des Spülkanals in den Raumbereich zum Aufnehmen des Arretierungssporns über, sodass in den ersten Abschnitt des Spülkanals eingeleitete Spülflüssigkeit teilweise in den Raumbereich zum Aufnehmen des Arretierungssporn übergehen und diesen durchströmen kann. Der Raumbereich zum Aufnehmen des Arretierungssporns ist zwar an der dem zweiten Griffschenkel zugewandten Seite des ersten Griffschenkels angeordnet und dort einer Verunreinigung in vergleichsweise geringerem Maße ausgesetzt, jedoch als Öffnung und aufgrund seiner Funktion doch für eine Verunreinigung anfällig. Ein Spülen dieses Raumbereichs, zumal von innen nach außen, ist deshalb vorteilhaft.

Bei einer Handhabungseinrichtung, wie sie hier beschrieben ist, leitet der Raumbereich zum Aufnehmen des Arretierungssporns insbesondere Spülflüssigkeit von dem Spülanschluss zu der Arretiereinrichtung.

Die Arretiereinrichtung ist insbesondere unmittelbar außerhalb des Raumbereichs zum Aufnehmen des Arretierungssporns angeordnet und wird so von aus dem Raumbereich austretendem Spülfluid umspült und gereinigt.

Eine Handhabungseinrichtung, wie sie hier beschrieben ist, umfasst insbesondere ferner eine Abschalteinrichtung, die manuell bewegbar ist zwischen einer Abschaltposition und einer Arbeitsposition und die derart mit der Arretiereinrichtung mechanisch gekoppelt ist, dass die Arretiereinrichtung ihre Löseposition einnimmt, wenn die Abschalteinrichtung ihre Abschaltposition einnimmt, und dass die Arretiereinrichtung manuell zwischen ihrer Arretierungsposition und ihrer Löseposition bewegbar ist, wenn die Abschalteinrichtung ihre Arbeitsposition einnimmt.

Die Abschalteinrichtung ist insbesondere mittelbar mit der Arretiereinrichtung mechanisch gekoppelt, beispielsweise über die Löseeinrichtung. Alternativ kann die Abschalteinrichtung unmittelbar mit der Arretiereinrichtung mechanisch gekoppelt sein, beispielsweise mittels einer Feder oder eines anderen elastischen oder starren Verbindungsbauteils.

Eine Handhabungseinrichtung, wie sie hier beschrieben ist, umfasst insbesondere ferner eine Abschalteinrichtung, die manuell bewegbar ist zwischen einer Abschaltposition und einer Arbeitsposition, und die derart mit der Löseeinrichtung mechanisch gekoppelt ist, dass die Löseeinrichtung ihre Löseposition einnimmt, wenn die Abschalteinrichtung ihre Abschaltposition einnimmt, und dass die Löseeinrichtung manuell zwischen ihrer Arretierungsposition und ihrer Löseposition bewegbar ist, wenn die Abschalteinrichtung ihre Arbeitsposition einnimmt.

Die mechanische Kopplung zwischen der Abschalteinrichtung und der Löseeinrichtung erfolgt beispielsweise mittels einer Feder oder eines anderen elastischen oder starren Verbindungsbauteils. In der Abschaltposition der Abschalteinrichtung hält die Feder die Löseeinrichtung in ihrer Löseposition. In der Arbeitsposition der Abschalteinrichtung übt die Feder keine oder nur eine geringe Kraft auf die Löseeinrichtung aus, sodass diese zwischen ihrer Arretierungsposition und ihrer Löseposition bewegbar ist.

Eine Handhabungseinrichtung, wie sie hier beschrieben ist, umfasst insbesondere ferner einen dritten Abschnitt des Spülkanals, der den Spülanschluss oder den ersten Abschnitt des Spülkanals mit der Abschalteinrichtung fluidisch verbindet.

Eine Handhabungseinrichtung, wie sie hier beschrieben ist, umfasst insbesondere ferner eine Abschalteinrichtung, die manuell bewegbar ist zwischen einer Abschaltposition und einer Arbeitsposition, und die derart mit der Arretiereinrichtung mechanisch gekoppelt ist, dass die Arretiereinrichtung ihre Löseposition einnimmt, wenn die Abschalteinrichtung ihre Abschaltposition einnimmt, und dass die Arretiereinrichtung manuell zwischen ihrer Arretierungsposition und ihrer Löseposition bewegbar ist, wenn die Abschalteinrichtung ihre Arbeitsposition einnimmt, und einen dritten Abschnitt des Spülkanals zum Zuführen von Spülfluid zu der Abschalteinrichtung.

Die Abschalteinrichtung ist insbesondere mittelbar, nämlich über die Löseeinrichtung mit der Arretiereinrichtung mechanisch gekoppelt. Wenn die Abschalteinrichtung in ihrer Abschaltposition ist, hält sie die Löseeinrichtung in ihrer Löseposition und damit mittelbar auch die Arretiereinrichtung in ihrer Löseposition.

Der dritte Abschnitt des Spülkanals verbindet insbesondere den Spülanschluss unmittelbar oder mittelbar über den ersten Abschnitt oder über den ersten Abschnitt und den zweiten Abschnitt mit der Abschalteinrichtung. Durch das Zuführen von Spülfluid zu der Abschalteinrichtung kann auch die Abschalteinrichtung durch das Spülfluid gereinigt werden.

Bei einer Handhabungseinrichtung, wie sie hier beschrieben ist, lenkt die Löseeinrichtung insbesondere durch den ersten Abschnitt des Spülkanals strömende Spülflüssigkeit abhängig von ihrer Position mehr oder weniger in den dritten Abschnitt des Spülkanals.

Die Lenkungswirkung der Löseeinrichtung beruht insbesondere auf einem mehr oder weniger vollständigen Verschließen oder Verlegen eines oder mehrerer alternativer Strömungspfade. Insbesondere verschließt die Löseeinrichtung abhängig von ihrer Position einen Ausgang, durch den durch den ersten Abschnitt des Spülkanals strömende Spülflüssigkeit aus der Handhabungseinrichtung austreten kann, mehr oder weniger, sodass die Spülflüssigkeit mehr oder weniger in den dritten Abschnitt des Spülkanals strömt.

Bei einer Handhabungseinrichtung, wie sie hier beschrieben ist, quert der dritte Abschnitt des Spülkanals insbesondere die Löseeinrichtung, wobei der Querschnitt der Löseeinrichtung im Bereich des dritten Abschnitts des Spülkanals schmaler ist als der Querschnitt der Löseeinrichtung in einem zur unmittelbaren manuellen Betätigung vorgesehenen Bereich.

Der dritte Abschnitt des Spülkanals quert die Löseeinrichtung insbesondere in einem Winkel zwischen 30 Grad und 90 Grad bezogen auf die Haupterstreckungsrichtung der Löseeinrichtung an dieser Stelle.

Die in Richtung orthogonal zu den Haupterstreckungsrichtungen der Arme der Löseeinrichtung und orthogonal zu dem dritten Abschnitt des Spülkanals gemessene Breite des Querschnitts der Löseeinrichtung ist im Bereich des dritten Abschnitts dessen Spülkanals kleiner als in einem zur unmittelbaren manuellen Betätigung vorgesehenen Bereich der Löseeinrichtung. Insbesondere ist die Breite des Querschnitts der Löseeinrichtung im Bereich des dritten Abschnitts um mindestens ein Viertel oder mindestens ein Drittel oder mindestens die Hälfte geringer als in einem zur unmittelbaren manuellen Betätigung vorgesehenen Bereich.

Durch die im Bereich des dritten Abschnitts des Spülkanals geringere Breite des Querschnitts der Löseeinrichtung kann Spülflüssigkeit an der Löseeinrichtung vorbei von einer Seite der Löseeinrichtung zu einer gegenüberliegenden Seite der Löseeinrichtung und damit insbesondere zu der Abschalteinrichtung strömen.

Bei einer Handhabungseinrichtung, wie sie hier beschrieben ist, ist der dritte Abschnitt des Spülkanals in dem Bereich, in dem er die Löseeinrichtung quert, insbesondere gegabelt und führt an zwei voneinander abgewandten Seiten der Löseeinrichtung vorbei.

Die gegabelte Ausbildung des dritten Abschnitts des Spülkanals kann eine vollständige Umspülung der Löseeinrichtung an dieser Stelle ermöglichen. Ferner kann die gegabelte Ausbildung des dritten Abschnitts des Spülkanals den für die Spülflüssigkeit zur Verfügung stehenden Gesamtquerschnitt vergrößern und damit den Strömungswiderstand reduzieren. Nach dem Passieren der Löseeinrichtung kann in dem dritten Abschnitt des Spülkanals strömendes Spülfluid wieder einen einzigen, einfach zusammenhängenden Querschnitt ausfüllen.

Eine Handhabungseinrichtung, wie sie hier beschrieben ist, umfasst insbesondere ferner einen Grundkörper, der einen Griffschenkel bildet und einen Hohlraum aufweist, und eine Tragstruktur, die in dem Hohlraum in dem Grundkörper angeordnet und mit dem Grundkörper mechanisch starr verbunden ist, wobei Wellen, die Schwenkachsen von zumindest entweder der Arretiereinrichtung oder der Löseeinrichtung oder der Abschalteinrichtung definieren, in der Tragstruktur befestigt oder geführt sind.

Eine Handhabungseinrichtung, wie sie hier beschrieben ist, umfasst insbesondere ferner einen Grundkörper, der einen Griffschenkel bildet und einen Hohlraum aufweist, und eine Tragstruktur, die in dem Hohlraum in dem Grundkörper angeordnet und mit dem Grundkörper mechanisch starr verbunden ist, wobei Gelenke, die Schwenkachsen von zumindest entweder der Arretiereinrichtung oder der Löseeinrichtung oder der Abschalteinrichtung definieren, in der Tragstruktur vorgesehen sind.

Eine Handhabungseinrichtung, wie sie hier beschrieben ist, umfasst insbesondere ferner einen Grundkörper, der einen Griffschenkel bildet und einen Hohlraum aufweist, und eine Tragstruktur, die in dem Hohlraum in dem Grundkörper angeordnet und mit dem Grundkörper mechanisch starr verbunden ist, wobei Wellen, die Schwenkachsen von zumindest entweder der Arretiereinrichtung oder der Löseeinrichtung oder der Abschalteinrichtung definieren, in der Tragstruktur befestigt oder geführt sind, wobei im Bereich des dritten Abschnitts des Spülkanals die Tragstruktur die Oberflächen des Hohlraums in den Grundkörpern nicht bedeckt.

Indem die Tragstruktur im Bereich des dritten Abschnitts des Spülkanals die Oberflächen des Hohlraums in dem Grundkörper nicht bedeckt, bleibt erst der Querschnitt des dritten Abschnitts des Spülkanals innerhalb des Hohlraums in dem Grundkörper frei.

Bei einer Handhabungseinrichtung, wie sie hier beschrieben ist, weist der Grundkörper an einer ersten Seite eine erste Öffnung, in der die Arretiereinrichtung angeordnet ist, und in die der Arretierungssporn eingreifen kann, zu dem Hohlraum hin auf, wobei der Grundkörper an einer von der ersten Seite abgewandten zweiten Seite eine zweite Öffnung, durch die die Löseeinrichtung hindurchgreift, zu dem Hohlraum hin und eine dritte Öffnung, durch die die Abschalteinrichtung hindurchgreift, zu dem Hohlraum hin aufweist, wobei ein Steg die zweite Öffnung und die dritte Öffnung trennt, und wobei der Steg teilweise den Querschnitt des dritten Abschnitts des Spülkanals begrenzt.

Der Steg leitet also in dem dritten Abschnitt das Spülfluid, so dass dieses vollständig oder weitgehend zu der Abschalteinrichtung geleitet wird. Ohne den Steg würden die zweite Öffnung und die dritte Öffnung ineinander übergehen, und Spielfluid könnte aus der Handhabungseinrichtung austreten, ohne seine volle Wirkung entfaltet zu haben.

Ein medizinisches Instrument umfasst eine Handhabungseinrichtung, wie sie hier beschrieben ist.

Das medizinische Instrument umfasst insbesondere ferner einen Schaft, der mit der Handhabungseinrichtung dauerhaft und nicht zerstörungsfrei lösbar verbunden sein kann. Alternativ kann der Schaft zerstörungsfrei, also reversibel und insbesondere auch ohne Verwendung von Werkzeug von der Handhabungseinrichtung trennbar und wieder mit dieser verbindbar sein. Das medizinische Instrument kann ferner ein Werkzeug am distalen Ende des Schafts aufweisen, beispielsweise ein Werkzug zum Greifen, Quetschen und/oder Schneiden. Das Werkzeug kann mit dem distalen Ende des Schafts dauerhaft und nicht zerstörungsfrei trennbar verbunden sein. Alternativ kann das Werkzeug zerstörungsfrei und insbesondere auch ohne Verwendung von Werkzeug reversibel von dem distalen Ende des Schafts trennbar und wieder mit diesem verbindbar sein.

### Kurzbeschreibung der Figuren

Nachfolgend werden Ausführungsformen anhand der beigefügten Figuren näher erläutert. Es zeigen:

| | |
|---|---|
| Figur 1 | eine schematische Darstellung eines mikroinvasiven medizinischen Instruments; |
| Figur 2 | eine schematische vergrößerte Darstellung einer Handhabungseinrichtung des Instruments aus Figur 1; |
| Figur 3 | eine schematische Darstellung eines Schnitts durch die Handhabungseinrichtung aus Figur 2; |
| Figur 4 | eine weitere schematische Darstellung eines Schnitts durch die Handhabungseinrichtung aus den Figuren 2 und 3; |
| Figur 5 | eine weitere schematische Darstellung eines Schnitts durch die Handhabungseinrichtung aus den Figuren 2 bis 4; |
| Figur 6 | eine weitere schematische Darstellung eines Schnitts durch die Handhabungseinrichtung aus den Figuren 2 bis 5; |
| Figur 7 | eine weitere schematische Darstellung eines Schnitts durch die Handhabungseinrichtung aus den Figuren 2 bis 6; |
| Figur 8 | eine weitere schematische Darstellung eines Schnitts durch die Handhabungseinrichtung aus den Figuren 2 bis 7. |

### Beschreibung der Ausführungsformen

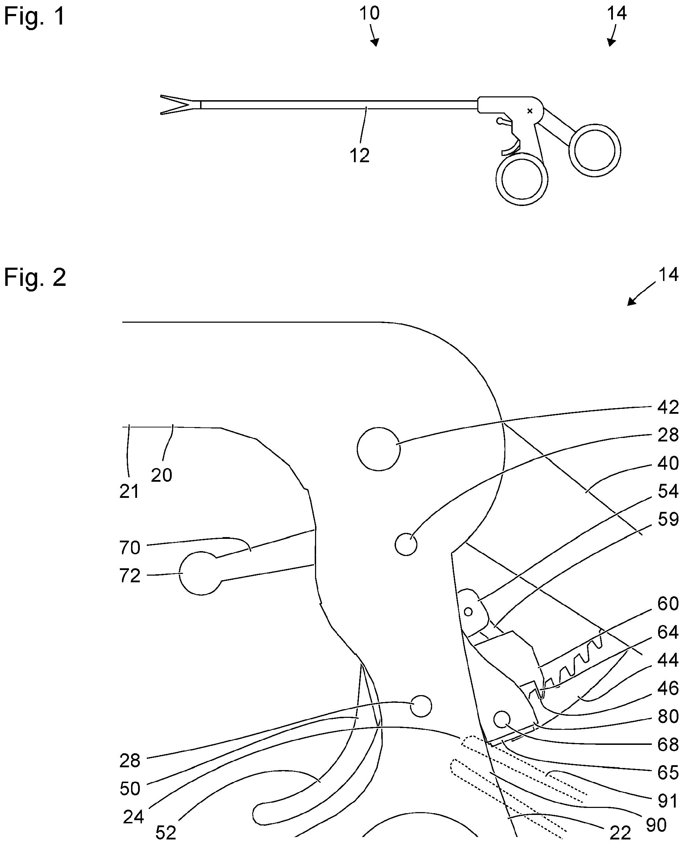

Figur 1 zeigt eine schematische Darstellung eines mikroinvasiven medizinischen Instruments 10 mit einem langen und dünnen Schaft 12 und einem Werkzeug an dem distalen Ende des Schafts 12, die teilweise oder vollständig in einen natürlichen oder künstlichen Hohlraum in einem Körper eines menschlichen oder tierischen Patienten eingeführt werden können. Am proximalen Ende des Schafts 12 ist eine Handhabungseinrichtung 14 vorgesehen, mittels derer das medizinische Instrument, vor allem das Werkzeug am distalen Ende des Schafts 12, manuell gesteuert werden kann. Die Handhabungseinrichtung 14 kann mit dem Schaft 12 derart verbunden sein, dass der Schaft 12 und die Handhabungseinrichtung 14 nicht zerstörungsfrei und reversibel trennbar sind. Alternativ ist das medizinische Instrument 10 beispielsweise so ausgebildet, dass der Schaft 12 und die Handhabungseinrichtung 14 ohne Verwendung von Werkzeug und zerstörungsfrei, das heißt reversibel voneinander getrennt und wieder miteinander verbunden werden können. Dazu kann beispielsweise eine Renkverbindung (oft auch als Bajonettverbindung bezeichnet) und/oder eine Rastverbindung vorgesehen sein.

Figur 2 zeigt eine schematische und gegenüber Figur 1 vergrößerte Darstellung der Handhabungseinrichtung 14. Die Handhabungseinrichtung 14 umfasst einen Grundkörper 20, der insbesondere ursprünglich einstückig gefertigt ist, beispielsweise durch ein Gussverfahren, ein spanendes Verfahren, 3D-Druck oder ein anderes additives Verfahren. Der Grundkörper kann aus chirurgischem Edelstahl oder einem anderen Metall, Keramik, Kunststoff oder einem oder mehreren anderen Materialien gebildet sein.

Der Grundkörper 20 weist einen distalen Bereich 21 auf, der mit dem Schaft 12 (vergleiche Figur 1) verbunden sein oder in diesen übergehen kann. Der Grundkörper 20 bildet ferner einen Griffschenkel 22, der beispielsweise ähnlich wie eine Schere ein Auge zum Aufnehmen von einem oder mehreren Fingern von medizinischem Personal aufweisen kann. Der Griffschenkel 22 ist gegenüber dem distalen Bereich 21 des Grundkörpers insbesondere um einen Winkel von circa 45 Grad bis 90 Grad, insbesondere 60 Grad bis 90 Grad, abgewinkelt.

Die Handhabungseinrichtung 14 weist ferner einen schwenkbaren Griffschenkel 40 auf, der gegenüber dem Grundkörper 20 um eine durch ein Gelenk 42 definierte Schwenkachse, die orthogonal zu der Zeichenebene der Figur 2 ist, schwenkbar ist. Ein Rastsporn 44 mit mehreren oder vielen Rastnuten 46 ist starr mit dem schwenkbaren Griffschenkel 40 verbunden.

Die Handhabungseinrichtung 14 weist ferner einen Lösehebel 50 auf. Ein erster Arm 52 des Lösehebels 50 ist zur unmittelbaren manuellen Betätigung vorgesehen und ausgebildet. Der erste Arm 52 des Lösehebels 50 ist an einer von dem zweiten Griffschenkel 40 abgewandten Seite des ersten Griffschenkels 22 angeordnet. Der Lösehebel 50 ist teilweise in einem Hohlraum in dem Grundkörper 30 angeordnet. Ein zweiter Arm 54 des Lösehebels 50 ist mit dessen erstem Arm 52 starr verbunden und mit diesem zusammen um ein Schwenkachse, die orthogonal zu der Zeichenebene der Figur 2 und durch ein in Figur 2 nicht sichtbares Schwenkgelenk definiert ist, schwenkbar. Der Lösehebel 50 weist einen zweiten Arm 54 auf, dessen von dem ersten Arm 52 abgewandtes Ende an einer dem zweiten Griffschenkel 40 zugewandten Seite des ersten Griffschenkels 22 angeordnet ist.

Der Lösehebel 50 ist durch ein Verbindungsbauteil 59 mit einer Arretiereinrichtung 60 mechanisch gekoppelt. Ein Ende des Verbindungsbauteils 59 ist durch ein Gelenk mit dem von dem ersten Arm 52 abgewandten Ende des zweiten Arms 54 verbunden. Das andere Ende des Verbindungsbauteils 59 ist durch ein weiteres Gelenk mit der Arretiereinrichtung 60 verbunden. Das Verbindungsbauteil 59 ist insbesondere im Wesentlichen stangen- oder stabförmig.

Die Arretiereinrichtung 60 umfasst eine Rastklinke 64 zum Eingreifen in eine beliebige der Rastnuten 46 des Rastsporns 44. Die Arretiereinrichtung 60 ist um eine durch ein Gelenk 68 definierte Schwenkachse, die orthogonal zu der Zeichenebene der Figur 2 ist, schwenkbar. In der in Figur 2 gezeigten Rastposition der Arretiereinrichtung 60 greift die Rastklinke 64 der Arretiereinrichtung 60 in eine der Rastnuten 46 des Rastsporns 44 ein und verhindert dadurch formschlüssig eine Vergrößerung des Winkels zwischen dem zweiten Griffschenkel 40 und dem ersten Griffschenkel 22. In einer in Figur 2 nicht gezeigten Löseposition der Arretiereinrichtung greift die Rastklinke 64 nicht in eine der Rastnuten 46 des Rastsporns 44 ein, und der Winkel zwischen dem zweiten Griffschenkel 40 und dem ersten Griffschenkel 22 kann verändert werden. Wie nachfolgend beschrieben kann durch manuelle Betätigung des ersten Arms 52 des Lösehebels 50 die Arretiereinrichtung 60 in ihre in Figur 2 nicht gezeigte Löseposition bewegt werden. Ferner sind die Rastklinke 64 der Arretiereinrichtung 60 und die Rastnuten 46 des Rastsporns 44 so ausgebildet, dass eine Verringerung des Winkels zwischen dem zweiten Griffschenkel 40 und dem ersten Griffschenkel 22 stets möglich ist, indem die Arretiereinrichtung 60 vorübergehend aus ihrer Rastposition in Richtung ihrer Löseposition ausweicht.

Die Handhabungseinrichtung 14 weist ferner einen Abschalthebel 70 auf, der ebenfalls teilweise in dem erwähnten Hohlraum in dem Grundkörper 30 angeordnet und dort mit dem Lösehebel 50 mechanisch gekoppelt ist. Der Abschalthebel 70 ist um Schwenkachse, die durch ein in Figur 2 nicht sichtbares Gelenk definiert und orthogonal zu der Zeichenebene der Figur 2 ist, schwenkbar zwischen einer Abschaltposition und einer Arbeitsposition (oder mehreren Arbeitspositionen, insbesondere einem Bereich von Arbeitspositionen).

Der Abschalthebel 70 ist mit dem Lösehebel 50 derart mechanisch gekoppelt, dass der Lösehebel 50 in seiner in Figur 2 nicht dargestellten Löseposition gehalten ist und damit mittelbar auch die Arretiereinrichtung in ihrer in Figur 2 nicht dargestellten Löseposition gehalten ist, wenn der Abschalthebel seine in Figur 2 nicht dargestellte Abschaltposition einnimmt. Der Abschalthebel 70 ist mit dem Lösehebel 50 derart mechanisch gekoppelt, dass der Lösehebel 50 zwischen seiner in Figur 2 gezeigten Rastposition und seiner in Figur 2 nicht dargestellten Löseposition und damit auch die Arretiereinrichtung 60 zwischen ihrer in Figur 2 gezeigten Rastposition und ihrer in Figur 2 nicht dargestellten Löseposition bewegt werden kann, wenn der Abschalthebel 70 seine Arbeitsposition einnimmt oder die Position des Abschalthebels 70 innerhalb des Bereichs von Arbeitspositionen liegt.

In dem erwähnten, jedoch in Figur 2 nicht sichtbaren Hohlraum 30 in dem Grundkörper 20 ist eine Tragstruktur 80 angeordnet, die im Bereich der Arretiereinrichtung 60 aus dem Hohlraum herausragt. Die Arretiereinrichtung 60 ist um eine Schwenkachse, die durch ein Gelenk 68 zwischen der Arretiereinrichtung 60 und der Tragstruktur 80 definiert und orthogonal zu der Zeichenebene der Figur 2 ist, schwenkbar.

Der in Figur 2 nicht sichtbare Hohlraum und die Tragstruktur 80 weisen insbesondere jeweils zwei einander gegenüberliegende ebene und parallele Oberflächenbereiche auf, wobei die äußeren Oberflächenbereiche der Tragstruktur 80 an den korrespondierenden inneren Oberflächenbereichen des Hohlraums anliegen. Passstifte 28, die sich orthogonal zu der Zeichenebene der Figur 2 erstrecken und deren Enden in Figur 2 sichtbar sind, fixieren die Tragstruktur 80 in dem Hohlraum.

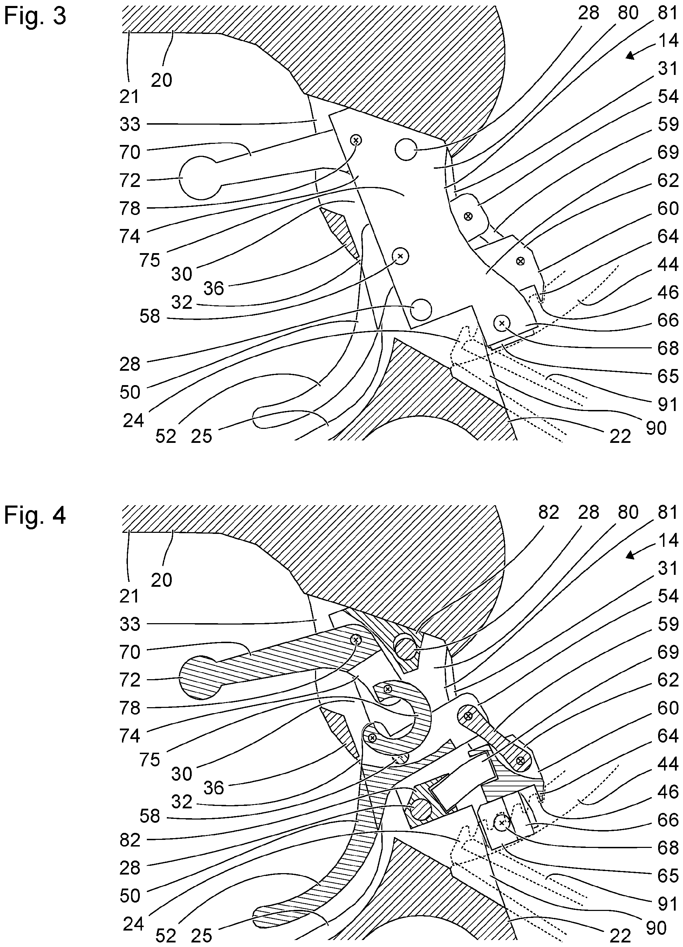

Figur 3 zeigt eine schematische Darstellung eines Schnitts durch den Grundkörper 20 der Handhabungseinrichtung 14 aus den Figuren 1 und 2. Der Grundkörper 20 ist entlang einer Schnittebene parallel zu der Zeichenebene der Figur 2 geschnitten dargestellt. Der schwenkbare zweite Griffschenkel 40 (vergleiche Figur 2) ist in Figur 3 nicht dargestellt. Auch Details seiner gelenkigen Verbindung mit dem Grundkörper 20 und seiner mechanischen Kopplung mit einer Zugstange und/oder anderen Einrichtungen des medizinischen Instruments sind in Figur 3 nicht dargestellt. In Figur 3 ist der bereits erwähnte Hohlraum 30 in dem Grundkörper 20 sichtbar. In dem Hohlraum 30 ist die Tragstruktur 80 angeordnet, die den Hohlraum 30 teilweise ausfüllt. Die Tragstruktur 80, der Lösehebel 50, die Arretiereinrichtung 60 und der Abschalthebel 70 sind in Figur 3 nicht im Schnitt, sondern in Draufsicht dargestellt.

Der Grundkörper 20 weist drei Öffnungen zu dem Hohlraum 30 hin auf. Eine erste Öffnung 31 zu dem Hohlraum 30 hin ist an der dem zweiten Griffschenkel 40 (vergleiche Figur 2) zugewandten Seite des ersten Griffschenkels 22 angeordnet. Der zweiter Arm 54 des Lösehebels 50 und die Tragstruktur 80 ragen durch die erste Öffnung aus dem Hohlraum 30 heraus. Die Arretiereinrichtung 60 ist weitgehend oder vollständig außerhalb des Hohlraums 30 angeordnet. Ein Teil des Hohlraums 30, der nicht durch die Tragstruktur 80 ausgefüllt wird, bildet einen zur Aufnahme des Rastsporns 44 vorgesehenen Raumbereichs 24. Ein Randbereich des Hohlraums 30 ist ferner als Spülanschluss 90 zur Aufnahme eines in Figur 3 in gestrichelter Linie angedeuteten Rohres 91 oder einer entsprechenden Tülle zur Zufuhr von Spülfluid vorgesehen und ausgebildet. Der Spülanschluss ist insbesondere als konkaver spitzwinkeliger Konusstumpf entsprechend dem Luer-Standard ausgebildet.

Der Grundkörper 20 weist ferner an seiner von dem zweiten Griffschenkel 40 (vergleiche Figur 2) abgewandten Seite eine zweite Öffnung 32 und eine dritte Öffnung 33 zu dem Hohlraum 30 hin auf. Die zweite Öffnung 32 und die dritte Öffnung 33 werden von einem Steg 36 getrennt. Der Lösehebel 50, nämlich sein erster, zur unmittelbaren manuellen Betätigung vorgesehener Arm, ragt durch die zweite Öffnung 32 aus dem Hohlraum 30 heraus. An die zweite Öffnung 32 schließt sich eine Nut 25 in den Grundkörper 20 an, die vorgesehen und ausgebildet ist, den ersten, unmittelbar manuell betätigbaren ersten Arm 52 des Lösehebels 50 teilweise aufzunehmen. Durch eine Linie ist eine Änderung des Querschnitts des ersten Arms 52 des Lösehebels 50 im Bereich der zweiten Öffnung 32 angedeutet. Der erste Arm 52 des Lösehebels 50 weist außerhalb des Hohlraums 30 eine größere in Richtung orthogonal zu der Zeichenebene der Figur 3 gemessene Breite auf als innerhalb.

Ein Gelenk 58 zwischen dem Lösehebel 50 und der Tragstruktur 80 definiert eine Schwenkachse, die orthogonal zu der Zeichenebene der Figur 3 ist. Das Gelenk 58 wird beispielsweise aus anhand der Figur 4 beschriebenen Gründen durch zwei kurze Wellen oder Stifte gebildet, die einerseits die vom Betrachter abgewandte Seite und andererseits die dem Betrachter zugewandte Seite des Lösehebels mit der Tragstruktur 80 verbinden.

Der Abschalthebel 70 ragt durch die dritte Öffnung 33 aus dem Hohlraum 30 heraus. Ein Gelenk 78 zwischen dem Abschalthebel 70 und der Tragstruktur 80 definiert eine Schwenkachse, die orthogonal zu der Zeichenebene der Figur 3 ist. Das Schwenkgelenk 78 ist durch eine Welle oder Achse gebildet, deren Ende in Figur 3 sichtbar ist.

Figur 4 zeigt eine schematische Darstellung eines Schnitts durch die Handhabungseinrichtung 14 aus den Figuren 1 bis 3. Die Position der Schnittebene der Figur 4 entspricht derjenigen der Schnittebene des Grundkörpers 20 in Figur 3. In Gegensatz zu Figur 3 sind jedoch in Figur 4 auch der Lösehebel 50, die Arretiereinrichtung 60, der Abschalthebel 70 und die Tragstruktur 80 im Schnitt entlang derselben Ebene dargestellt.

Die Tragstruktur 80 umfasst zwei Seitenteile bzw. -teilbereiche 81, die parallel zu der Schnittebene der Figur 4 angeordnet sind, und von denen in Figur 4 nur eine, nämlich die hinter der Schnittebene liegende sichtbar ist. Von einander abgewandte Oberflächenbereiche der Seitenteile bzw. -teilbereiche 81 liegen flächig an der inneren Oberfläche des Hohlraums Die Tragstruktur 80 umfasst ferner zwei Pfeiler 82 mit unregelmäßigen Querschnitten, welche die Seitenteile bzw. -teilbereiche 81 mechanisch starr verbinden. Die Passstifte 28, die die Tragstruktur 80 in dem Hohlraum 30 fixieren, sind innerhalb der Pfeiler 82 angeordnet.

Der zweite Arm 54 des Lösehebels 50 ist teilweise gegabelt bzw. mit einer tiefen Längsnut parallel zu der Schnittebene der Figur 4 ausgebildet. In dieser Längsnut sind ein Ende des Verbindungsbauteils 59 und ein Teil einer C-förmigen Feder 75 angeordnet. Da die durch das Schwenkgelenk 58 definierte Schwenkachse des Lösehebels 50 diese Längsnut durchdringt, ist das Schwenkgelenk 58 durch zwei kurze Wellen beiderseits dieser Längsnut, das heißt vor bzw. hinter der Schnittebene der Figur 4, gebildet.

Die Arretiereinrichtung 60 weist eine sich parallel zu der Schnittebene der Figur 4 erstreckende Nut 65 zur Aufnahme des Rastsporns 44 auf. Das Schwenkgelenk 68 ist im Bereich dieser Nut 65 angeordnet, die durch das Schwenkgelenk 68 definierte Schwenkachse durchdringt die Nut 65 orthogonal. Deshalb ist das Gelenk 68 wie oben erwähnt durch zwei kurze Wellen oder Stifte gebildet, die beiderseits dieser Nut 65, das heißt vor bzw. hinter der Schnittebene der Figur 4, die Arretiereinrichtung 60 mit der Tragstruktur 80 verbinden.

Die Arretiereinrichtung 60 weist ferner eine Nut 62 parallel zu der Schnittebene der Figur 4 auf, in der ein Ende des Verbindungsbauteils 59 angeordnet ist.

Die Enden einer Feder 69 sind in einer Tasche in einem der Pfeiler 82 der Tragstruktur 80 bzw. in einer Tasche in der Arretiereinrichtung 60 angeordnet. Die Feder 69 ist in Figur 4 nur durch ihre Umrisse angedeutet und tatsächlich insbesondere als komprimierte Schraubenfeder ausgebildet.

In Figur 4 sind der Abschalthebel 70 in einer Arbeitsposition, der Lösehebel 50 in seiner Arretierungsposition und die Arretiereinrichtung 60 in ihrer Arretierungsposition gezeigt.

Ein von dem manuell betätigbaren Bereich 72 des Abschalthebels 70 abgewandtes Ende 74 des Abschalthebels 70 ist durch die C-förmige Feder 75 mit dem Lösehebel 50 gekoppelt.

Figur 5 zeigt eine schematische Darstellung eines weiteren Schnitts durch die Handhabungseinrichtung 14 aus den Figuren 1 bis 4. Die Art der Darstellung, insbesondere die Lage der Schnittebene entspricht derjenigen der Figur 4.

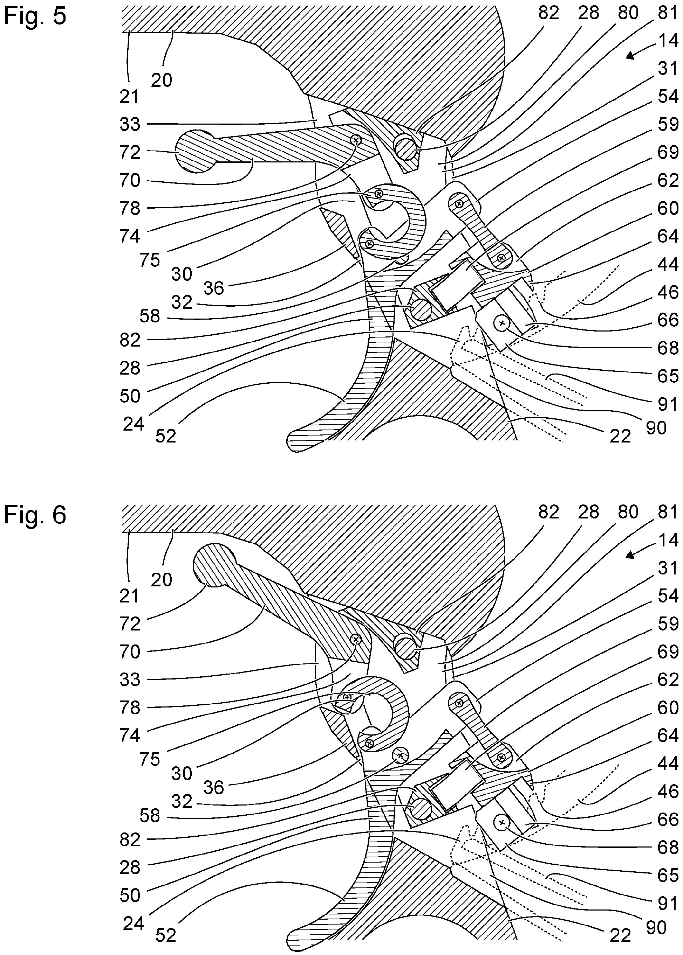

Wie bei der anhand der Figuren 2 bis 4 dargestellten Situation oder Konfiguration befindet sich der Abschalthebel 70 auch in der in Figur 5 gezeigten Situation oder Konfiguration in einer Arbeitsposition. Die C-förmige Feder 75 zwischen dem Abschalthebel 70 und dem Lösehebel 50 erzwingt keine besondere Position des Lösehebels. Die in Figur 5 gezeigte Situation oder Konfiguration unterscheidet sich von der anhand der Figuren 2 bis 4 dargestellten Konfiguration jedoch dadurch, dass der Lösehebel 50 durch unmittelbare manuelle Betätigung, nämlich Drücken seines ersten Arms 52 zu dem ersten Griffschenkel 22 hin bewegt ist und seine Löseposition in der Nut 25 (vergleiche Figuren 3, 4) einnimmt. Durch die mechanische Kopplung mittels des Verbindungsbauteils 59 nimmt bei dieser Löseposition des Lösehebels 50 auch die Arretiereinrichtung 60 eine Löseposition ein, bei der die Rastklinke 64 den Arretierungssporn 44 nicht arretiert.

Bei der in Figur 5 gezeigten Löseposition der Arretiereinrichtung 60 ist die Feder 69 zwischen der Arretiereinrichtung 60 und der Tragstruktur 80 gegen ihre Rückstellkraft komprimiert. Deshalb bewegt die elastische Rückstellkraft der Feder 69 die Arretiereinrichtung 60 und damit auch den Lösehebel 50 ausgehend von ihren in Figur 5 gezeigten Lösepositionen wieder in ihre in Figur 4 gezeigten Rastpositionen, wenn der Lösehebel 50 nicht manuell gegen die Rückstellkraft der Feder 69 zu der in Figur 5 gezeigten Löseposition gedrückt wird.

Figur 6 zeigt eine schematische Darstellung eines weiteren Schnitts durch die Handhabungseinrichtung 14 aus den Figuren 2 bis 5. Die Art der Darstellung, insbesondere die Schnittebene, entspricht derjenigen der Figuren 4 und 5.

In Figur 6 ist der Abschalthebel 70 in einer Abschaltposition gezeigt, in der die C-förmige Feder 75 den Lösehebel 50 in seiner Löseposition und damit mittelbar die Arretiereinrichtung 60 in ihrer Löseposition hält. Dazu ist die C-förmige Feder 75 zwischen dem Abschalthebel 70 und dem Lösehebel 50 so dimensioniert, dass das von ihr auf den Lösehebel 50 ausgeübte Drehmoment größer ist als das von der Feder 69 vermittels des Verbindungsbauteils 59 auf den Lösehebel 50 ausgeübte Drehmoment umgekehrten Vorzeichens. Die C-förmige Feder 75 hält ferner den Abschalthebel 70 in seiner in Figur 6 gezeigten Abschaltposition.

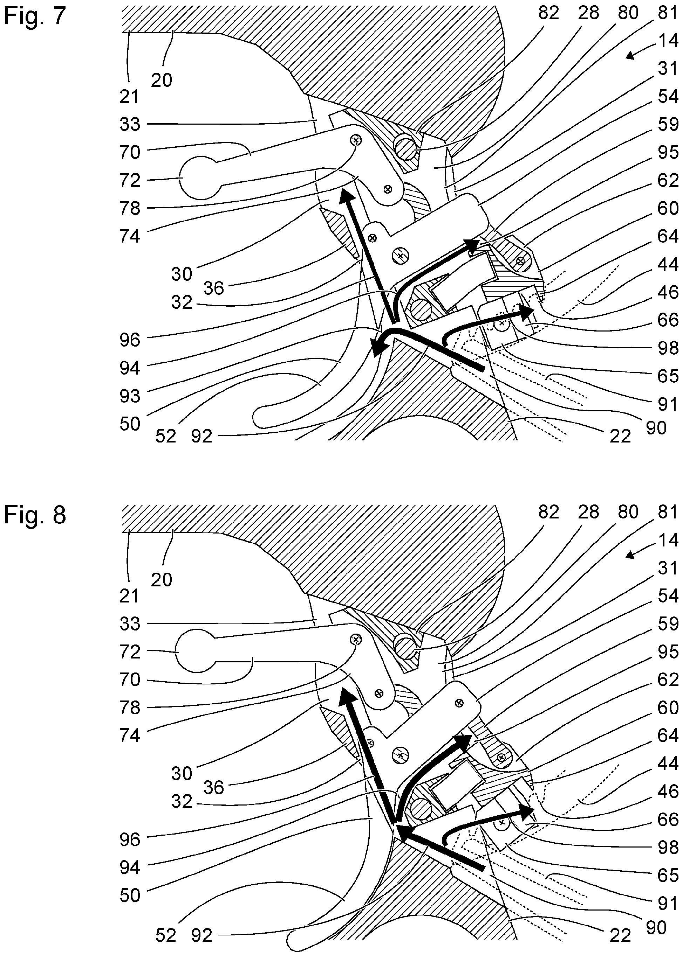

Figur 7 zeigt eine weitere Darstellung eines Schnitts durch die Handhabungseinrichtung 14 aus den Figuren 2 bis 6. Die Art der Darstellung entspricht weitgehend derjenigen der Figuren 4 bis 6, wobei jedoch der Lösehebel 50 und der Abschalthebel 70 nicht im Schnitt dargestellt sind.

In Figur 7 ist die bereits anhand der Figur 4 dargestellte Konfiguration gezeigt, bei der der Abschalthebel 70 eine Arbeitsposition und der Lösehebel 50 und die Arretiereinrichtung 60 jeweils ihre Arretierungspositionen einnehmen. In Figur 7 sind mehrere Abschnitte 92, 94, 96, 98 eines verzweigten Spülkanals zum Spülen und Reinigen der Handhabungseinrichtung 14 mittels eines Spülfluids angedeutet. Die Abschnitte 92, 94, 96, 98 des Spülkanals sind durch Pfeile angedeutet, die die Strömungsrichtung des Spülfluids andeuten.

Ein erster Abschnitt 92 des Spülkanals führt ausgehend von dem Spülanschluss 90 im Wesentlichen geradeaus an der Tragstruktur 80 vorbei zu einem Ausgang 93, an dem das Spülfluid teilweise durch die Nut 25 und zwischen dem ersten Griffschenkel 22 und dem ersten Arm 52 des Lösehebels 50 aus der Handhabungseinrichtung 14 austreten kann.

Ein Teil des Spülfluids fließt jedoch innerhalb der Handhabungseinrichtung 14 weiter durch einen zweiten Abschnitt 94 des Spülkanals zwischen dem zweiten Arm 54 des Lösehebels 50 und einem Pfeiler 82 der Tragstruktur 80 zu dem Verbindungsbauteil 59 und einem von der Rastklinke 64 abgewandten Bereich der Arretiereinrichtung 60, um dort aus der Handhabungseinrichtung 14 auszutreten. Dabei bildet der Bereich zwischen den Rändern des Seitenteils bzw. -teilbereichs 81, dem zweiten Arm 54 des Lösehebels, der Arretiereinrichtung 60 und der Verbindungseinrichtung 60 den Ausgang 95 des zweiten Abschnitts 94 des Spülkanals.

Ein weiterer Teil des Spülfluids strömt entlang eines dritten Abschnitts 96 beiderseits (das heißt bezogen auf Figur 7 vor und hinter) dem Lösehebel 50 vorbei zu dem Abschalthebel 70. Der dritte Abschnitt 96 des Spülkanals wird teilweise begrenzt von dem Steg 36 und den gegenüberliegenden Rändern der Seitenteile bzw. -teilbereiche 81 der Tragstruktur 80. Der dritte Abschnitt 96 des Spülkanals führt zu der dritten Öffnung 33 des Hohlraums 30, die überwiegend als Ausgang für das Spülfluid fungiert.

Ein vierter Abschnitt 98 des Spülkanals zweigt von dem ersten Abschnitt 92 ab und führt durch den zur Aufnahme des Rastsporns 44 (vergleiche Figur 2) vorgesehenen Raumbereich, durch die Nut 65 in der Arretiereinrichtung 60 für die Aufnahme des Rastsporns 44 zu der Rastklinke 64 und verlässt dort die Handhabungseinrichtung. Die Nut 65 zur Aufnahme des Rastsporns 44 in der Arretiereinrichtung 60 ist hier gleichzeitig der Ausgang des vierten Abschnitts 98 des Spülkanals.

Figur 8 zeigt eine weitere schematische Darstellung eines Schnitts durch die Handhabungseinrichtung aus den Figuren 2 bis 7. Die Art der Darstellung entspricht derjenigen der Figur 7.

Die in Figur 8 gezeigte Konfiguration entspricht der in Figur 5 gezeigten Konfiguration, bei der der Abschalthebel 70 eine Arbeitsposition und der Lösehebel 50 und die Arretiereinrichtung 60 ihre Lösepositionen einnehmen. In seiner in Figur 8 gezeigten Löseposition verschließt der Lösehebel 50 den unmittelbaren Ausgang 93 des ersten Abschnitts 92 des Spülkanals zumindest weitgehend, sodass deutlich mehr Spülflüssigkeit in den zweiten Abschnitt 94, in den dritten Abschnitt 96 und in den vierten Abschnitt 98 des Spülkanals gelangen. Dies ist durch veränderte Breiten der die Abschnitte 94, 96 repräsentierenden Pfeile angedeutet.

### Bezugszeichen

- **10**: **Medizinisches Instrument**
- 12: Schaft des medizinischen Instruments 10
- 14: Handhabungseinrichtung des medizinischen Instruments 10
- **20**: **Grundkörper** der Handhabungseinrichtung 20
- 21: distaler Bereich des Grundkörpers 30
- 22: Griffschenkel des Grundkörpers 30
- 24: Raumbereich zur Aufnahme des Rastsporns 44
- 25: Nut zur Aufnahme des manuell betätigbaren Bereichs 52 des Lösehebels 50
- 28: Stift zur Fixierung der Tragstruktur 80 in dem Grundkörper 20
- **30**: **Hohlraum** in dem Grundkörper 30
- 31: erste Öffnung zu dem Hohlraum 30
- 32: zweite Öffnung zu dem Hohlraum 30
- 33: dritte Öffnung zu dem Hohlraum 30
- 36: Steg zwischen der zweiten Öffnung 32 und der dritten Öffnung 33
- **40**: **schwenkbarer Griffschenkel** der Handhabungseinrichtung
- 42: Gelenk zwischen dem schwenkbaren Griffschenkel 40 und dem Grundkörper 20
- 44: Rastsporn an dem schwenkbaren Griffteil 40
- 46: Rastnut in dem Rastsporn 44
- **50**: **Lösehebel** der Handhabungseinrichtung 20
- 52: erster Arm des Lösehebels 50, unmittelbar manuell betätigbar
- 54: zweiter Arm des Lösehebels 50, von dem ersten Arm 52 abgewandt und mit der Arretiereinrichtung 60 durch das Verbindungsbauteil 59 mechanisch gekoppelt
- 58: Welle, ein Schwenkgelenk zwischen Lösehebel 50 und Tragstruktur 80 bildend und eine Schwenkachse des Lösehebels 50 definierend
- 59: Verbindungsbauteil zur mechanischen Kopplung des Lösehebels 50 mit der Arretiereinrichtung 60
- **60**: **Arretiereinrichtung** der Handhabungseinrichtung 20, mit dem Lösehebel 50 mechanisch gekoppelt
- 62: Nut in der Arretiereinrichtung 60, zur Aufnahme eines Endes des Verbindungsbauteils 59
- 64: Rastklinke der Arretiereinrichtung 60, zum Eingreifen in eine Rastnut 46 im Rastsporn 44
- 65: Nut zur Aufnahme des Rastsporns 44
- 66: Anlaufschräge zur Zentrierung des Rastsporns 44
- 68: Welle, ein Schwenkgelenk zwischen Arretiereinrichtung 60 und Tragstruktur 80 bildend und eine Schwenkachse der Arretiereinrichtung 60 definierend
- 69: Feder zwischen der Arretiereinrichtung 60 und der Tragstruktur 80
- **70**: **Abschalthebel** der Handhabungseinrichtung
- 72: manuell betätigbarer Bereich des Abschalthebels 70
- 74: von dem manuell betätigbaren Bereich 72 abgewandtes Ende des Abschalthebels 70, mit dem Lösehebel 50 durch die Feder 75 mechanisch gekoppelt
- 75: Feder zur mechanischen Kopplung des Abschalthebels 70 mit dem Lösehebel 50
- 78: Welle, ein Schwenkgelenk zwischen Abschalthebel 70 und Tragstruktur 80 bildend und eine Schwenkachse des Abschalthebels 70 definierend
- **80**: **Tragstruktur** in dem Hohlraum 30
- 81: Seitenteil bzw. -teilbereich der Tragstruktur
- 82: Pfeiler zwischen den Seitenteilen bzw. -teilbereichen 81
- **90**: **Spülanschluss** der Handhabungseinrichtung 14
- 91: Rohr zum Zuführen von Spülfluid
- 92: erster Abschnitt eines Spülkanals
- 93: Ausgang des ersten Abschnitts 92 des Spülkanals
- 94: zweiter Abschnitt des Spülkanals
- 95: Ausgang des zweiten Abschnitts 94 des Spülkanals
- 96: dritter Abschnitt des Spülkanals
- 98: vierter Abschnitt des Spülkanals

## Patentansprüche

1. **Handhabungseinrichtung** (14) für ein medizinisches Instrument (10), mit:
einem **ersten Griffschenkel** (22) und einem **zweiten Griffschenkel** (40), die relativ zu einander bewegbar sind;
einem **Spülanschluss** (90) zum Empfangen einer Spülflüssigkeit zur Reinigung der Handhabungseinrichtung (14) zwischen zwei Verwendungen;
einem **Spülkanal** (92, 94, 96, 98), der den Spülanschluss (90) mit einem oder mehreren Ausgängen (93, 95, 33, 65) verbindet,
wobei der **Spülkanal** (92, 94, 96, 98) **in dem ersten Griffschenkel** (22) angeordnet ist.

2. Handhabungseinrichtung (14) gemäß dem vorangehenden Anspruch, bei der
der **Spülanschluss** (90) an einer dem zweiten Griffschenkel (40) zugewandten Seite des ersten Griffschenkels (22) angeordnet ist.

3. Handhabungseinrichtung (14) gemäß einem der vorangehenden Ansprüche, bei der
der **Spülanschluss** (90) **proximal** eines Verbindungsbereichs (42) der Griffschenkel (22, 40) angeordnet ist.

4. Handhabungseinrichtung (14) gemäß einem der vorangehenden Ansprüche, bei der
der zweite Griffschenkel (40) relativ zu dem ersten Griffschenkel (22) in einer Ebene bewegbar ist,
der **Spülkanal** (92, 94, 96, 98) im Wesentlichen **parallel zu der Ebene** verläuft.

5. Handhabungseinrichtung (14) gemäß einem der vorangehenden Ansprüche, ferner mit:
einem **Arretierungssporn** (44), der mit dem zweiten Griffschenkel (40) mechanisch starr verbunden oder gekoppelt ist, so dass eine Bewegung des zweiten Griffschenkels (40) relativ zu dem ersten Griffschenkel (22) mit einer Bewegung des Arretierungssporns (44) relativ zu dem ersten Griffschenkel (22) einhergeht;
einer **Arretiereinrichtung** (60), die mit dem ersten Griffschenkel (22) mechanisch verbunden und relativ zu dem ersten Griffschenkel (22) bewegbar ist zwischen einer Löseposition ohne Wechselwirkung mit dem Arretierungssporn (44) und einer Arretierungsposition, in der die Arretiereinrichtung (64) form- oder kraftschlüssig mit dem Arretierungssporn (44) verbunden sein kann;
einer **Löseeinrichtung** (50), die manuell bewegbar ist zwischen einer Löseposition und
einer Arretierungsposition, und die derart mit der Arretiereinrichtung (60) mechanisch gekoppelt ist, dass die Arretiereinrichtung (60) ihre Löseposition einnimmt, wenn die Löseeinrichtung (50) ihre Löseposition einnimmt, und dass die Arretiereinrichtung (60) ihre Arretierungsposition einnimmt, wenn die Löseeinrichtung (50) ihre Arretierungsposition einnimmt.

6. Handhabungseinrichtung (14) gemäß dem vorangehenden Anspruch, bei der
ein **erster Abschnitt** (92) des Spülkanals den Spülanschluss (90) mit der **Löseeinrichtung** (50) verbindet.

7. Handhabungseinrichtung (14) gemäß dem vorangehenden Anspruch, bei der
der erste Abschnitt (92) des Spülkanal den Spülanschluss (90) mit einer **Ausnehmung** (25), die die Löseeinrichtung (50) in ihrer Löseposition zumindest teilweise aufnimmt, verbindet.

8. Handhabungseinrichtung (14) gemäß einem der Ansprüche 6 und 7, bei der
die Löseeinrichtung (50) abhängig von ihrer Position durch den ersten Abschnitt (92) des Spülkanals strömende Spülflüssigkeit mehr oder weniger **in einen zweiten Abschnitt** (94) des Spülkanals lenkt,
ein Querschnitt des zweiten Abschnitts (94) des Spülkanals teilweise durch die Löseeinrichtung (50) begrenzt ist.

9. Handhabungseinrichtung (14) gemäß dem vorangehenden Anspruch, bei der
der zweite Abschnitt (94) des Spülkanals Spülflüssigkeit **zu einem Verbindungsbauteil** (59), das den zweiten Arm (54) der Löseeinrichtung (50) mit der Arretiereinrichtung (60) mechanisch koppelt oder verbindet, leitet.

10. Handhabungseinrichtung (14) gemäß einem der Ansprüche 6 bis 9, bei der
der Spülanschluss (90) ferner mit einem Raumbereich (24) zum Aufnehmen des **Arretierungssporns** (44) fluidisch verbunden ist.

11. Handhabungseinrichtung (14) gemäß einem der Ansprüche 5 bis 10, ferner mit:
einer **Abschalteinrichtung** (70), die manuell bewegbar ist zwischen einer Abschaltposition und einer Arbeitsposition, und die derart mit der Arretiereinrichtung (60) mechanisch gekoppelt ist, dass die Arretiereinrichtung (60) ihre Löseposition einnimmt, wenn die Abschalteinrichtung (70) ihre Abschaltposition einnimmt, und dass die Arretiereinrichtung (60) manuell zwischen ihrer Arretierungsposition und ihrer Löseposition bewegbar ist, wenn die Abschalteinrichtung (70) ihre Arbeitsposition einnimmt,
einem **dritte Abschnitt** (96) des Spülkanals, zum Zuführen von Spülfluid zu der Abschalteinrichtung (70).

12. Handhabungseinrichtung (14) gemäß dem vorangehenden Anspruch, bei der
die Löseeinrichtung (50) durch den ersten Abschnitt (92) des Spülkanals strömende Spülflüssigkeit abhängig von ihrer Position mehr oder weniger **in den dritten Abschnitt** (96) des Spülkanals lenkt.

13. Handhabungseinrichtung (14) gemäß einem der Ansprüche 11, 12, bei der
der dritte Abschnitt (96) des Spülkanals die Löseeinrichtung (50) quert,
der Querschnitt der Löseeinrichtung (50) im Bereich des dritten Abschnitts (96) des Spülkanals schmaler ist als der Querschnitt der Löseeinrichtung (50) in einem zur unmittelbaren manuellen Betätigung vorgesehenen Bereich (52).

14. Handhabungseinrichtung (14) gemäß dem vorangehenden Anspruch, bei der
der dritte Abschnitt (96) des Spülkanals in dem Bereich, in dem er die Löseeinrichtung (50) quert, gegabelt ist und an zwei von einander abgewandten Seiten der Löseeinrichtung (50) vorbei führt.

15. Handhabungseinrichtung (14) gemäß einem der vorangehenden Ansprüche, mit:
einem **Grundkörper** (20), der einen Griffschenkel (22) bildet und einen Hohlraum (30) aufweist;
einer **Tragstruktur** (80), die in dem Hohlraum (30) in dem Grundkörper (20) angeordnet und mit dem Grundkörper (20) mechanisch starr verbunden ist, wobei **Wellen** (58, 68, 78), die Schwenkachsen von zumindest entweder der Arretiereinrichtung (60) oder der Löseeinrichtung (50) oder der Abschalteinrichtung (70) definieren, in der Tragstruktur (80) befestigt oder geführt sind,
wobei im Bereich des **dritten Abschnitts** (96) des Spülkanals die **Tragstruktur** (80) **die Oberflächen des Hohlraums** (30) in dem Grundkörper (20) **nicht bedeckt**.

16. Handhabungseinrichtung (14) gemäß dem vorangehenden Anspruch, bei der
der Grundkörper (20) an einer ersten Seite eine **erste Öffnung** (31), in der die Arretiereinrichtung (60) angeordnet ist und die der Arretierungssporn (44) eingreifen kann, zu dem Hohlraum (30) hin aufweist,
der Grundkörper (20) an einer von der ersten Seite abgewandten zweiten Seite eine **zweite Öffnung** (32), durch die die Löseeinrichtung (50) hindurchgreift, zu dem Hohlraum (30) hin und eine **dritte Öffnung** (33), durch die die Abschalteinrichtung (70) hindurchgreift, zu dem Hohlraum (30) hin aufweist,
ein **Steg** (36) die zweite Öffnung (32) und die dritte Öffnung (33) trennt,
der Steg (36) teilweise den Querschnitt des **dritten Abschnitts** (96) des Spülkanals **begrenzt**.
